# EUROPÄISCHE PATENTSCHRIFT

(11) **EP 2 262 456 B1**
(45) Veröffentlichungstag und Bekanntmachung des Hinweises auf die Patenterteilung: **04.04.2012**
(21) Anmeldenummer: 09720057.0
(22) Anmeldetag: 27.02.2009
(51) Int. Cl.: A61F 5/058

(54) **STÜTZSCHALENANORDNUNG ZUR ANORDNUNG AN EINEM UNTERSCHENKEL**
SUPPORT SHELL ARRANGEMENT FOR ARRANGING ON A LOWER LEG
DISPOSITIF DE COQUE DE SOUTIEN, POUR MISE EN PLACE CONTRE UNE JAMBE

(30) Priorität: 10.03.2008 DE 102008013382
(43) Veröffentlichungstag der Anmeldung: 22.12.2010
(73) Patentinhaber: OPED AG, 6330 Cham (CH)
(72) Erfinder: HOPMANN, Gero, 85579 Neubiberg (DE)
(74) Vertreter: Böck, Bernhard
(86) Internationale Anmeldenummer: PCT/EP2009/001392
(87) Internationale Veröffentlichungsnummer: WO 2009/112164

(56) Entgegenhaltungen:
- EP-A- 1 561 439
- DE-A1- 10 163 706
- US-A- 5 176 623

## Beschreibung

Die vorliegende Erfindung betrifft eine Stützschalenanordnung zur Anordnung an einem Unterschenkel mit einem Fußteil und einem Wadenteil nach dem Oberbegriff des Anspruchs 1. Das Dokument DE-A-101 63 706 stellt der nächsten Stand der Technik dar.

Stützschalenanordnungen der oben genannten Art sind in unterschiedlichen Ausführungsformen bekannt, die sich im Wesentlichen dadurch unterscheiden, wie die für die Stützschalenfunktion notwendige Stabilität konstruktiv erreicht wird. Grundsätzlich kann man dabei unterscheiden zwischen einer "geschlossenen" Stützschalenanordnung, bei der die notwendige Stabilität durch eine den Unterschenkel und zumindest den angrenzenden Fußbereich vollumfänglich umschließende Schalenanordnung erreicht wird, und einer "offenen" Stützschalenanordnung, bei der die Stabilität der Stützschalen durch eine insbesondere am Wadenteil ausgebildete vollflächige Armierung der Schalenoberfläche hergestellt wird. Hierzu werden bekannte Materialien, wie beispielsweise Faserverstärkungen, eingesetzt.

Wenn zwischen den Schalenteilen der Stützschalenanordnung noch eine Gelenkfunktion realisiert werden soll, werden ergänzend zu den üblicherweise für die Schale verwendeten Kunststoffmaterialien metallische Strukturteile verwendet, um für eine Aufnahme der Stützschalenkräfte ausreichend steife bzw. biegefeste Gelenkbeschläge zu ermöglichen.

Insbesondere bei der Verwendung von Metall-Beschlagteilen an Stützschalenanordnungen hat es sich als nachteilig herausgestellt, dass es zu Röntgenuntersuchungen der in den Stützschalenanordnungen aufgenommenen Körperteile wegen der Undurchlässigkeit der Metallteile für die Röntgenstrahlung regelmäßig notwendig ist, die Stützschalenanordnung vom Köperteil zu entfernen. Weiterhin hat es sich in der Praxis sowohl hinsichtlich des Tragekomforts als auch aus hygienischen Gesichtspunkten als vorteilhaft herausgestellt, Stützschalenanordnungen möglichst luftdurchlässig herzustellen.

Der vorliegenden Erfindung liegt daher die Aufgabe zugrunde, eine Stützschalenanordnung vorzuschlagen, die einerseits eine möglichst luftdurchlässige, also gut belüftbare, Struktur sowie einen hohen Bedienungs- und Tragekomfort aufweist und andererseits mit einer hinreichenden Steifigkeit ausgestattet ist, um ohne die Verwendung metallener Tragstrukturteile die Ausbildung einer gelenkigen Verbindung zwischen dem Wadenteil und dem Fußteil zu ermöglichen.

Zur Lösung dieser Aufgabe weist die erfindungsgemäße Stützschalenanordnung die Merkmale des Anspruchs 1 auf.

Erfindungsgemäß weist das Wadenteil der Stützschalenanordnung zwei seitlich einer Einstiegsöffnung des Wadenteils angeordnete, in Längsrichtung des Wadenteils verlaufende Wadenstreben und eine in einem Strebenanschluss mit den Wadenstreben verbundene und zu einem Fersenteil des Fußteils hin verlaufende Fersenstrebe auf, und das Fußteil der Stützschalenanordnung ist mit einer Stützbügelanordnung versehen mit zwei sich U-förmig um einen Fußsohlenbereich herum erstreckenden Bügelstreben, deren freie Bügelenden paarweise in einem gemeinsamen Drehgelenk auf einer der Fußgelenkachse entsprechenden Gelenkachse drehgelenkig an eine Wadenstrebe angeschlossen sind. Weiterhin sind die Bügelstreben V-förmig zueinander angestellt und über eine gemeinsame Stützbasis miteinander verbunden, wobei die Stützbasis an ihrem fersenseitigen Ende gelenkig mit der Fersenstrebe des Wadenteils verbunden ist.

Aufgrund der erfindungsgemäßen Gestaltung der Stützschalenanordnung ist diese luftdurchlässig oder transparent aufgebaut, wobei die Struktur so gewählt ist, dass ohne die Verwendung von metallenen Struktur- oder Gelenkbeschlagteilen eine Gelenkfunktion zwischen dem Wadenteil und dem Fußteil realisierbar ist. Die erfindungsgemäße Stützschalenanordnung ist aufgrund des Verzichts auf metallene Beschlag- oder Strukturteile für Röntgenstrahlung transparent, so dass eine Entfernung der Stützschalenanordnung von einer Extremität zur Durchführung einer Röntgenuntersuchung nicht notwendig ist. Darüber hinaus ermöglicht die erfindungsgemäße Stützschalenanordnung trotz des Verzichts auf metallene Beschlag- oder Strukturteile die Ausbildung einer "offenen" Stützschalenanordnung mit den damit einhergehenden Vorteilen hinsichtlich des Trage- und Bedienkomforts. Insbesondere entfällt die bei "geschlossenen" Stützschalenanordnungen notwendige Kombination mit einer abdeckenden "Ergänzungsschale", also einer Schienbeinschale zur Abdeckung einer Wadenschale und einer Fußspannschale zur Abdeckung einer Fußschale, um nach dem Einstieg in die Stützschalenanordnung die Einstiegsöffnung zur Erzielung einer hinreichenden Stabilität der Stützschalenanordnung zu verschließen.

Als besonders vorteilhaft erweist es sich, wenn die Stützbasis der Stützbügelanordnung an ihrer Unterseite mit einer Verbindungseinrichtung zur Verbindung mit einer Sohleneinrichtung versehen ist, so dass über die Stützbasis eine möglichst unmittelbare Einleitung der Stützkräfte in die Sohleneinrichtung möglich ist.

Wenn die Stützbasis der Stützbügelanordnung auf ihrer Unterseite mit einer Behelfssohle versehen ist, kann in einer Minimalkonfiguration der Stützschalenanordnung bereits mit einem kleinstmöglichen Bauteileeinsatz eine voll funktionsfähige Stützschalenanordnung realisiert sein.

Wenn darüber hinaus bereits die Verbindungseinrichtung der Stützbügelanordnung als Behelfssohleneinrichtung ausgebildet ist, kommt der Verbindungseinrichtung eine im Hinblick auf eine Teilereduktion vorteilhafte Doppelfunktion zu.

Eine besonders einfach zu realisierende konstruktive Möglichkeit zur Überwachung bzw. Anzeige der im Einsatz der Stützschalenanordnung auftretenden Stützkräfte wird möglich, wenn die Stützbasis der Stützbügelanordnung auf ihrer Unterseite mit einer Auslöseeinrichtung für einen Kraftsensor versehen ist.

Besonders vielfältige und von der stabilisierenden Stützfunktion unbeeinflusste Gestaltungsmöglichkeiten des Fußteils ergeben sich, wenn die Stützbügelanordnung als ein von einer Inlayschale des Fußteils unabhängiges Bauteil ausgebildet ist.

Eine Kombination der Stützbügelanordnung mit der Inlayschale lässt sich besonders einfach und belastbar vermittels einer Rastverbindung realisieren.

Unabhängig von der gewählten Ausführung der Verbindung zwischen der Stützbügelanordnung und der Inlayschale ist es besonders vorteilhaft, wenn diese Verbindung lösbar gestaltet ist, beispielsweise um eine standardisierte Stützbügelanordnung mit individuell gestalteten Inlayschalen auf wechselnde Nutzer anpassen zu können.

Eine besonders komfortable Anpassung an den jeweiligen Nutzer wird auch möglich, wenn das Fußteil ein mit der Inlayschale kombinierbares Zehenteil aufweist.

Insbesondere eine längsverschiebbar ausgebildete Verbindung erweist sich für die praktische Handhabung als vorteilhaft.

Wenn das Zehenteil eine gegenüber einem Basisteil zur Verbindung mit der Inlayschale vermittels einer Gelenkeinrichtung verschwenkbare Zehenauflage aufweist, können definierte Zehenbeugewinkel vorgegeben werden, um im Bedarfsfall eine weitere Erhöhung des Tragekomforts oder eine schmerzlindernde oder heilungsfördernde Zehenbeugestellung vorzugeben.

Eine vorteilhafte Einstellung eines Fußbeugewinkels wird möglich, wenn zur fersenseitigen Verbindung der Stützbügelanordnung des Fußteils mit der Fersenstrebe des Wadenteils eine Beugewinkel-Einstelleinrichtung vorgesehen ist, mit einer an einem Anschlussende der Stützbügelanordnung angelenkten Lasche, die über einen Führungseingriff an einer an der Fersenstrebe ausgebildeten Führungsschiene in veränderbaren Positionen an der Fersenstrebe fixierbar ist.

Darüber hinaus ist auch die Einstellung eines Beugewinkelbereichs möglich, wenn die Lasche einen Laschenanschlag aufweist, der zur beidseitigen Begrenzung eines Verschiebewegs mit zwei in ihrer Relativposition gegenüber der Führungsschiene veränderbar an der Führungsschiene fixierbaren Schienenanschlägen zusammenwirkt.

Eine sowohl abstützende als auch den Wadenmuskel massierende Wirkung lässt sich erzielen, wenn das Wadenteil eine sich vom Strebenanschluss in einen wadenseitigen Zwischenraum der Wadenstreben erstreckende Wadenstütze aufweist, die sich federnd am Strebenanschluss abstützt.

Um bei gleichzeitig federnder Wirkung auch definierte Stützstellungen vorgeben zu können, ist es vorteilhaft, wenn die Wadenstütze an ihrem freien Endbereich über längsverschiebbar angeschlossene Anschläge mit den Wadenstreben verbunden ist.

Die Anschläge können in unterschiedlichen Positionen an den Wadenstreben fixierbar sein, um Grundeinstellungen vornehmen zu können, die eine Anpassung an den Wadenumfang des Nutzers ermöglichen.

Nachfolgend wird eine bevorzugte Ausführungsform der Stützschalenanordnung anhand der Zeichnung näher erläutert.

Es zeigen:
- **Fig. 1**: eine Stützschalenanordnung in perspektivischer Darstellung;
- **Fig. 2**: die in **Fig. 1** dargestellte Stützschalenanordnung mit von einem Fußteil entfernter Sohleneinrichtung;
- **Fig. 3**: eine Inlayschale der in **Fig. 1** dargestellten Stützschalenanordnung mit abgenommener Stützbügelanordnung;
- **Fig. 4**: eine Rückansicht der in **Fig. 1** dargestellten Stützschalenanordnung mit einer zwischen einem Wadenteil und einem Fußteil angeordneten Beugewinkel-Einstelleinrichtung;
- **Fig. 5**: die Stützschalenanordnung mit einem am Fußteil angeordneten Zehenteil;
- **Fig. 6**: die Stützschalenanordnung mit Unteransicht des Fußteils;
- **Fig. 7**: die Stützschalenanordnung mit einem zwischen dem Fußteil und der Sohleneinrichtung angeordneten Kraftsensor;
- **Fig. 8**: die Stützschalenanordnung mit einer Anschlageinrichtung für eine am Wadenteil angeordnete Wadenstütze;

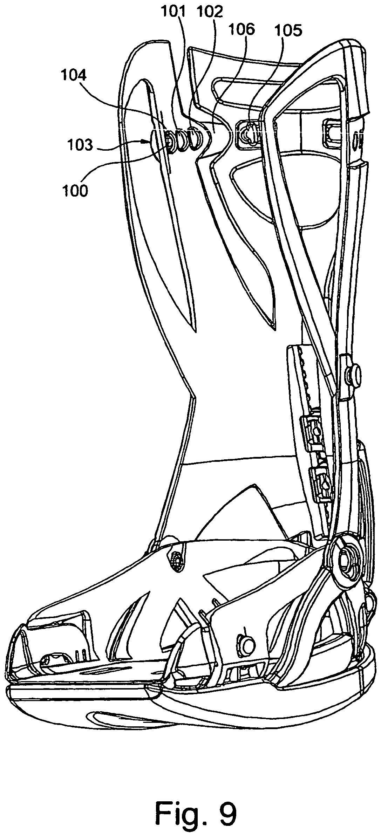

- **Fig. 9**: eine Innenansicht des Wadenteils;
- **Fig. 10**: die Stützschalenanordnung in Kombination mit einer Einstiegsöffnungs-Abdeckung;
- **Fig. 11**: eine weitere Möglichkeit der Anordnung eines Kraftsensors.

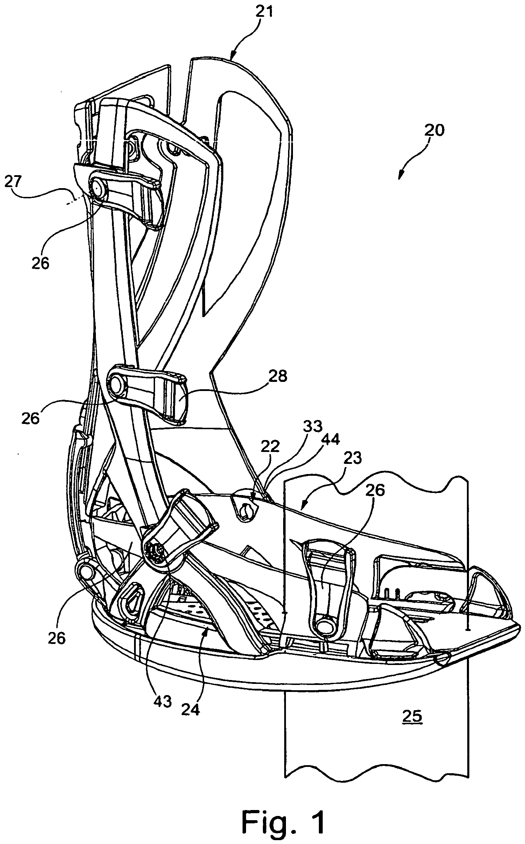

**Fig. 1** zeigt eine Stützschalenanordnung 20 mit einem Wadenteil 21 zur Aufnahme eines Unterschenkels und einem über eine Gelenkanordnung 22 mit dem Wadenteil 21 verbundenen Fußteil 23. Das Fußteil 23 ist mit einer Stützbügelanordnung 24 versehen, die ebenfalls über die Gelenkanordnung 22 mit dem Wadenteil 21 gelenkig verbunden ist.

Zur kraftschlüssigen Anordnung der Stützschalenanordnung 20 am Unterschenkel eines Patienten sind sowohl am Wadenteil 21 als auch am Fußteil 23 beidseitig einer in **Fig. 1** angedeuteten Fußmittelebene 25 **Gurtlaschen** 26 vorgesehen, die aus Gründen einer übersichtlichen Darstellung nur diesseits der Fußmittelebene dargestellt sind, sich jedoch spiegelbildlich zur Fußmittelebene 25 auch jenseitig dieser befinden. Die Gurtlaschen 26 sind um eine Laschenachse 27 verschwenkbar mit dem Wadenteil 21 bzw. dem Fußteil 23 verbunden und weisen an ihrem freien Ende im vorliegenden Fall eine Gurtöse 28 zur Durchführung eines hier nicht näher dargestellten Befestigungsgurtes auf.

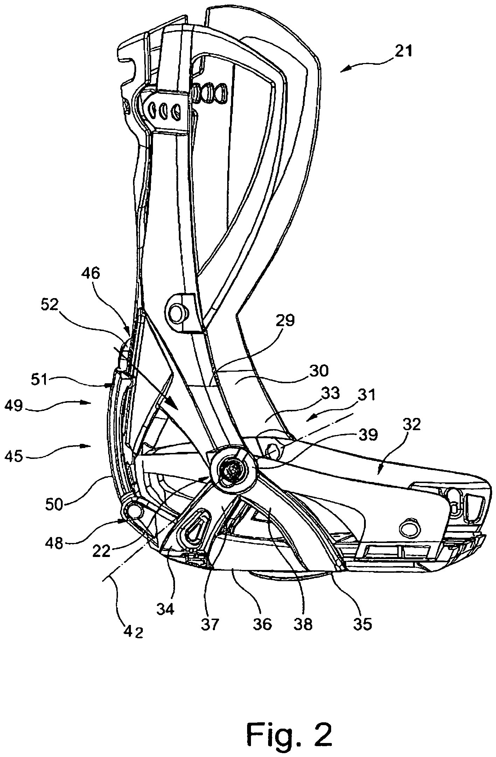

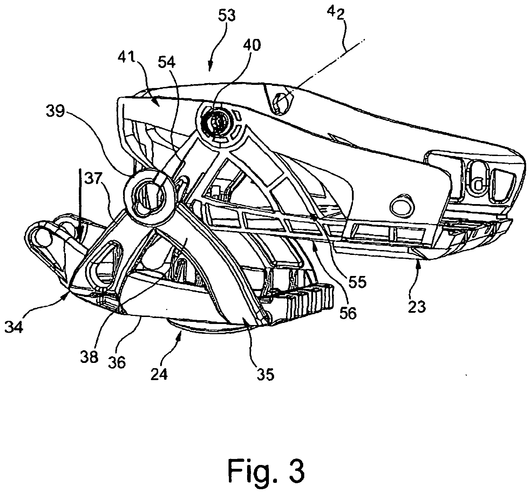

Wie insbesondere **Fig. 2** zeigt, weist das Wadenteil 21 als kraftleitende Strukturelemente zwei Wadenstreben 29, 30 auf, die zumindest in einem Fußknöchelbereich 31 eine Einstiegsöffnung 32 seitlich begrenzen. Die **Wadenstreben** 29, 30 verlaufen in Längsrichtung des Wadenteils 21 und münden mit ihren unteren Gelenkanschlussenden 33 in die Gelenkanordnung 22. Wie ebenfalls aus der **Fig. 2** zu ersehen ist, weist die Stützbügelanordnung 24 zwei U-förmig ausgebildete Bügelstreben 34, 35 auf, die über eine gemeinsame Bügelbasis 36 miteinander verbunden sind und mit ihren Bügelenden 37, 38 V-förmig zueinander angestellt in eine Gelenköse 39 münden. Wie aus einer Zusammenschau der **Fig. 2** und 3 zu ersehen ist, bildet die Stützbügelanordnung 24 über eine Eingriffsverbindung mit Gelenkzapfen 40 einer Inlayschale 53, die zur Anordnung in den Gelenkösen 39 dienen, das Fußteil 23.

Bei der in **Fig. 2** dargestellten Gelenkanordnung 22 sind hier nicht näher dargestellte, an den Gelenkanschlussenden 33 (**Fig**. **1**) der Wadenstreben 29, 30 ebenfalls ausgebildete Gelenkösen zwischen einer Seitenwand 41 der Inlayschale 53 und der Gelenköse 39 der Stützbügelanordnung 24 auf dem Gelenkzapfen 40 der Inlayschale 53 angeordnet, so dass über die Gelenkanordnung 22 das Fußteil 23, die Stützbügelanordnung 24 und das Wadenteil 21 in Drehgelenken 43, 44 auf einer gemeinsamen Gelenkachse 42, die im Wesentlichen parallel zu einer hier nicht näher dargestellten Fußknöchelachse verläuft, angeordnet sind.

Infolge der Anlenkung der Wadenstreben 29, 30 des Wadenteils 21 und der Bügelstreben 34, 35 der Stützbügelanordnung 24 werden über die Gelenkanordnung 22 von den Wadenstreben 29, 30 in die Gelenkanordnung 22 eingeleitete Zug- oder Druckkräfte in die Bügelstreben 34, 35 übertragen. Aufgrund der großen V-förmigen Anordnung der Bügelstreben 34, 35 werden dabei unabhängig davon, ob über die Wadenstreben 29, 30 Druck- oder Zugkräfte in die Gelenkanordnung 22 bzw. die Stützbügelanordnung 24 eingeleitet werden, Biegebeanspruchungen oder Knickbeanspruchungen der Bügelstreben 34, 35 verhindert. Aufgrund dieser wechselseitigen oder gegenseitigen Abstützung der Bügelstreben 34, 35 in der Gelenkanordnung 22 wird insbesondere die Gefahr, dass eine durch die Wadenstreben 29, 30 auf die Gelenkanordnung 22 ausgeübte Druckbelastung zu einer Destabilisierung der Stützschalenanordnung 20 durch eine Aufweitung der Stützschalenanordnung 20 durch axiales Auseinaderdriften der Drehgelenke 43, 44 auf der Gelenkachse 42 führt, weitestgehend vermieden. Die V-förmige Anordnung der Bügelstreben 34, 35 führt somit zu einer bezogen auf die Gelenkachse 42 axialen Aussteifung der Stützschalenanordnung 20.

Wie weiterhin aus der **Fig. 2** zu ersehen ist, erstreckt sich von einem oberhalb eines Fersenbereichs 45 gelegenen, die Wadenstreben 29, 30 miteinander verbindenden Strebenanschluss 46 eine Fersenstrebe 47 zu einem an der Stützbügelanordnung 24 ausgebildeten Fersenteil 48. Die Verbindung der Fersenstrebe 47 mit dem Fersenanschluss 48 erfolgt über eine Beugewinkel-Einstelleinrichtung 49, die mit einer gelenkig am Fersenanschluss 48 angelenkten Gelenklasche 50 über eine verstellbare Eingriffseinrichtung 51 mit einer an der Fersenstrebe 47 ausgebildeten Führungsschiene 52 verbunden ist.

**Fig. 3** zeigt in einer Explosionsdarstellung die Kombination der Stützbügelanordnung 24 mit der die Stützbügelanordnung 24 zum Fußteil 23 ergänzenden Inlayschale 53. Wie vorstehend bereits ausgeführt, ist die Inlayschale 53 zum einen über die in die an der Stützbügelanordnung 20 ausgebildeten Gelenkösen 39 eingreifenden Gelenkzapfen 40 kraftschlüssig mit der Stützbügelanordnung 24 verbunden. Darüber hinaus weist die Inlayschale 53 jeweils den Bügelstreben 34, 35 der Stützbügelanordnung 24 zugeordnete Schalenstreben 54, 55 auf, die sich von einem Schalenboden 56 im Wesentlichen deckungsgleich mit den Bügelstreben 34 und 35 zum Gelenkzapfen 40 hin erstrecken. Die Schalenstreben 54, 55 sind ebenso wie der Schalenboden 56 mit hier nicht näher dargestellten Rasteinrichtungen versehen, die mit an den Bügelstreben 34 und 35 sowie an der Bügelbasis 36 ausgebildeten, hier ebenfalls nicht näher dargestellten Rasteinrichtungen zusammenwirken und mit diesen eine Rastverbindung herstellen, wenn die Inlayschale 53 und die Stützbügelanordnung 24 so ineinander geführt werden, dass die Gelenkzapfen 40 der Inlayschale 53 in die Gelenkösen 39 der Stützbügelanordnung 24 eingreifen. Das Einsetzen der Gelenkzapfen 40 in die Gelenkösen 39 wird möglich durch ein Zusammendrücken der Seitenwände 41 der Inlayschale 53 in Richtung der Gelenkachse 42. Nach einem Lösen des Drucks werden dann aufgrund der elastischen Rückstellkräfte der Seitenwände 41 die Gelenkzapfen 40 in die Gelenkösen 39 eingeführt.

Aufgrund der vorstehend beschriebenen Kombination der relativ biegeweich ausgeführten Inlayschale 53 mit der relativ biegestarr ausgeführten Stützbügelanordnung 24 und den dabei hergestellten Rastverbindungen der ineinander greifenden Rasteinrichtungen sowie der in die Gelenkösen 39 der Stützbügelanordnung 24 eingreifenden Gelenkzapfen 40 der Inlayschale 53 wird ein biegestarrer Verbund geschaffen, der die Steifigkeit der Stützbügelanordnung 24 noch erhöht. Darüber hinaus bietet die von der im Wesentlichen die mechanische Stützfunktion erfüllenden Stützbügelanordnung 24 unabhängige Gestaltung der Inlayschale 53 die Möglichkeit, diese individuell an die Fußgeometrie des Nutzers anzupassen, wohingegen die Stützbügelanordnung 24 mit standardisierten Abmessungen und in standardisierter Geometrie ausführbar ist.

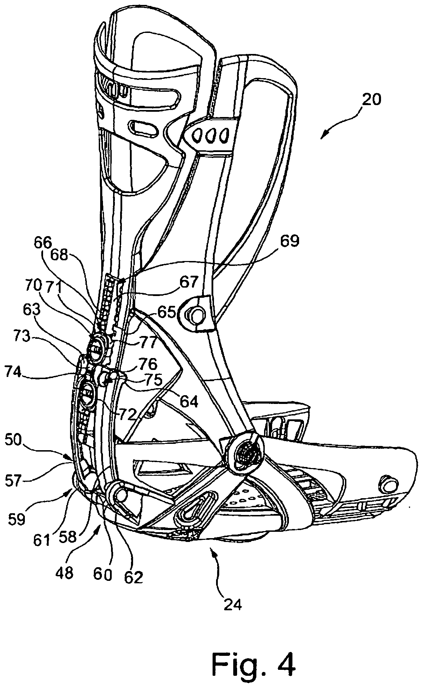

**Fig. 4** zeigt die Beugewinkel-Einstelleinrichtung 49 in einer Rückansicht der Stützschalenanordnung 20. Die Gelenklasche 50 der Beugewinkel-Einstelleinrichtung 49 ist im vorliegenden Fall U-förmig ausgeführt und über eine an den freien Enden von Laschenbügeln 57, 58 jeweils ausgebildete Gelenkeinrichtung 59, 60 drehgelenkig mit am Fersenanschluss 48 der Stützbügelanordnung 24 ausgebildeten Gelenkkonsolen 61, 62 verbunden. Die Laschenbügel 57, 58 gehen von einer gemeinsamen Laschenbasis aus, die einen Laschenanschlag 63 bildet. Im Bereich des Laschenanschlags 63 sind die Laschenbügel 57, 58 mit einem Führungszapfen 64 versehen, der jeweils einen Führungssteg 65 einer an der Fersenstrebe 47 ausgebildeten Führungsschiene 66 hintergreift.

Die Führungsschiene 66 ist mit einer sich in Längsrichtung der Führungsschiene 66 erstreckenden Eingriffsnut 67 versehen, die an ihren einander gegenüberliegenden Nuträndern 68 mit einer Eingriffsverzahnung 69 versehen ist. In die Eingriffsnut 67 sind lösbar und über eine Drehverriegelung 70 fixierbar Eingriffsriegel 71 und 72 eingesetzt. Die Eingriffsriegel 71, 72 liegen bei der in **Fig. 4** gewählten Darstellung mit Anschlagstücken 73, 74 einander gegenüberliegend am Laschenanschlag 63 an, so dass eine Bewegung des Laschenanschlags 63 längs der Führungsschiene 66 weder in die eine noch in die andere axiale Richtung möglich ist. In der in **Fig. 4** dargestellten Konfiguration fixieren die Eingriffsriegel 71 und 72 eine durch die Relativstellung des Laschenanschlags 63 gegenüber der Führungsschiene 66 definierte Beugewinkeleinstellung.

Um die Einstellung eines definierten Beugewinkels zu erleichtern, ist die Gelenklasche 50 im Bereich des Laschenanschlags 63 mit einem Sichtfenster 75 versehen, das die Sicht auf eine auf der Führungsschiene 66 vorgesehene Winkelmarkierung 76 frei gibt.

Die Eingriffsriegel 71, 72 sind an ihrem Umfang mit einer zur Eingriffsverzahnung 69 komplementär ausgebildeten Eingriffseinrichtung 77 versehen, die ein Einsetzen der Eingriffsriegel 71, 72 nach Maßgabe der durch die Eingriffsverzahnung 69 vorgegebenen Eingriffsteilung an beliebigen Stellen der Eingriffsnut 67 ermöglicht. Somit ist es auch möglich, die Eingriffsriegel 71, 72 abweichend von der in **Fig. 4** gewählten Darstellung mit größerem axialen Abstand voneinander in die Eingriffsnut 67 einzusetzen, um keinen definierten Beugewinkel, sondern einen Beugewinkelbereich zu definieren, der durch die Anschlagstücke 73, 74 der Eingriffsriegel 71, 72 begrenzt wird. Innerhalb dieses Anschlagbereichs kann der Laschenanschlag 63 längs der Führungsschiene 66 axial hin und her bewegt werden, so dass das Fußteil 23 gegenüber dem Wadenteil 21 innerhalb des entsprechenden Beugewinkelbereichs verschwenkbar ist.

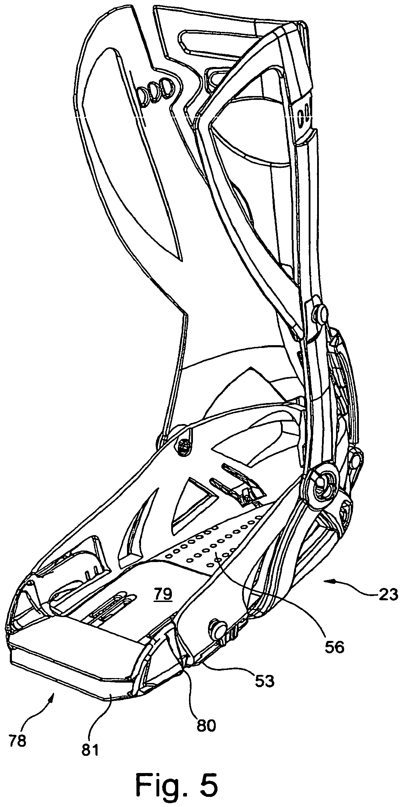

Wie **Fig. 5** zeigt, ist es möglich, das Fußteil 23 durch Kombination der Inlayschale 53 mit einem Zehenteil 78 zu versehen, das über eine relativ gegenüber dem Schalenboden 56 der Inlayschale 53 längs verschiebbare und am Schalenboden 56 über eine nicht näher dargestellte Eingriffsverbindung gesicherte Basisplatte 79 verfügt. Die Basisplatte 79 ist an ihrem zehenseitigen Rand über ein hier als Filmscharnier ausgebildetes Drehgelenk 80 mit einer Zehenplatte 81 versehen. Wie in **Fig. 5** durch den strichpunktierten Linienverlauf der Kontur der Zehenplatte 81 angedeutet, sind infolge der veränderbaren Relativstellung der Zehenplatte 81 gegenüber der Basisplatte 79 unterschiedliche Zehenbeugewinkel möglich. Im Bedarfsfall ist es auch möglich, die Zehenplatte 81 mit einer Fixierungsmöglichkeit zu versehen, um einen definierten Zehenbeugewinkel einzustellen.

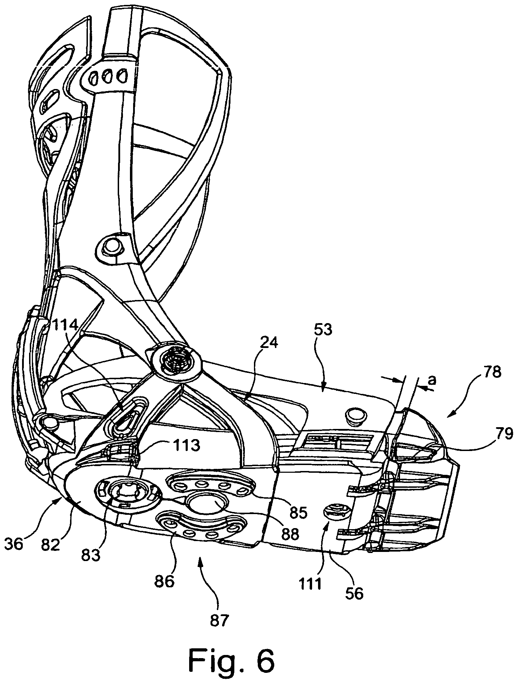

Wie **Fig. 6** zeigt, ist die Basisplatte 79 am Schalenboden 56 der Inlayschale 53 über eine lösbare Verriegelungseinrichtung 111 fixiert, die eine definierte Einstellung einer Ausfahrlänge a des Zehenteils 78 gegenüber der Inlayschale 53 ermöglicht, um eine Anpassung an individuelle Fußlängen vornehmen zu können.

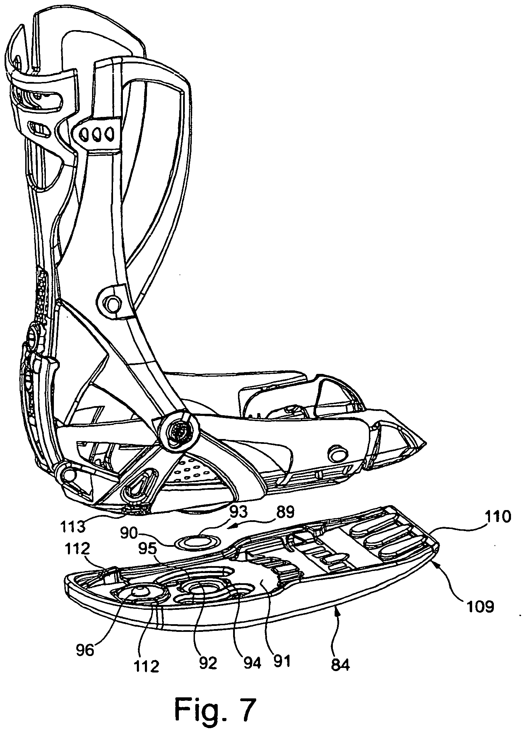

Wie weiterhin der **Fig. 6** zu entnehmen ist, ist die Bügelbasis 36 der Stützbügelanordnung 24 auf ihrer Unterseite 82 mit einer Anschlusseinrichtung 83 zur Verbindung der Bügelbasis 36 mit weiteren Anbauteilen, wie beispielsweise einer hier nicht näher dargestellten Stützeinrichtung zur positionierenden Lagerung der Stützschalenanordnung, versehen. Weiterhin weist gemäß **Fig. 6** die Bügelbasis 36 auf ihrer Unterseite 82 eine hier aus zwei Sohlenteilen 85, 86 gebildete Behelfssohleneinrichtung 87 auf, die unter bestimmten Bedingungen auch eine Nutzung der Stützschalenanordnung 20 ohne die in **Fig. 7** dargestellte Sohleneinrichtung 84 ermöglichen soll.

Wie aus einer Zusammenschau der **Fig. 6** und 7 ersichtlich wird, ist auf der Unterseite 82 der Bügelbasis 36 zwischen den Sohlenteilen 85 und 86 eine konvexe Druckfläche 88 vorgesehen, die als Auslöser für einen hier als Federring 89 ausgebildeten Kraftsensor dient. Der Federring 89 liegt mit einer als ebener Außenring 90 ausgebildeten Auflagefläche auf einer auf einer Montageseite 91 der Sohleneinrichtung 84 ausgebildeten ringförmigen Auflagefläche 92 auf. Bei einem ausreichend hohen, durch die konkave Druckfläche 88 auf einen inneren Ringkonus 93 des Federrings 89 ausgeübten Druck schlägt der Ringkonus 93 in Richtung auf die Auflagefläche 92 verbunden mit einen Knackgeräusch um, so dass bei entsprechender Abstimmung der Federkonstante des Federrings 89 das Knackgeräusch das Überschreiten einer definierten Grenz- bzw. Gewichtskraft anzeigt und somit eine Überlastwarnung ermöglicht.

Weiterhin sind auf der Montageseite 91 der Sohleneinrichtung 84 Aufnahmen 94, 95 für die Sohlenteile 85, 86 der Behelfssohleneinrichtung 87 ausgebildet. Zur positionsgenauen Verbindung der Bügelbasis 36 mit der Sohleneinrichtung 84 ist auf der Montageseite 91 der Sohleneinrichtung 84 ein Zentrierzapfen 96 ausgebildet. Zusätzlich sorgt eine formschlüssige Relativausrichtung der Sohlenteile 85, 86 der Notlaufsohleneinrichtung 87 mit den in der Sohleneinrichtung 84 ausgebildeten Aufnahmen 94, 95 für eine zum Eindringen des Zentrierzapfens 96 in die Anschlusseinrichtung 83 der Bügelbasis 36 günstige Relativausrichtung. Die Herstellung der Verbindung der Sohleneinrichtung 84 mit der Bügelbasis 36 erfolgt nach Art einer "step in"-Verbindung, derart, dass beim Einführen des Zentrierzapfens 96 in die Anschlusseinrichtung 83 eine Verrastung von an der Sohleneinrichtung 84 ausgebildeten Rastnasen 112 mit an der Bügelbasis ausgebildeten, auslenkbaren Rastriegeln 113 erfolgt. Ein Auslenken der Rastriegel 113 zum Lösen der Verbindung kann über Drucktasten 114 erfolgen.

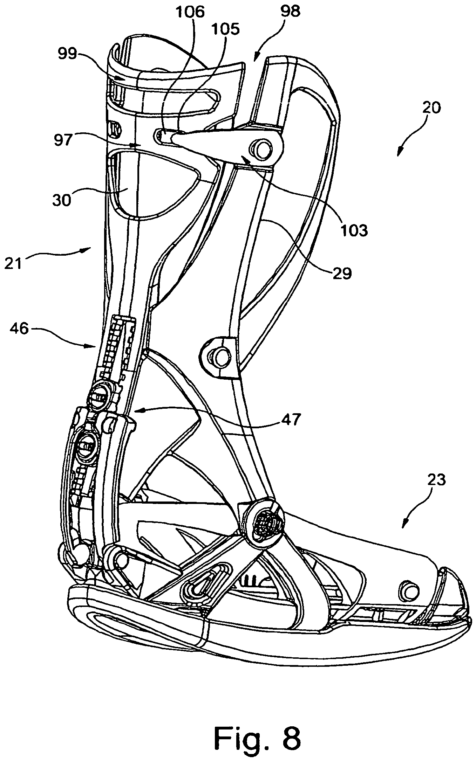

Aus der in **Fig. 8** dargestellten Rückansicht der Stützschalenanordnung 20 ist zu ersehen, dass das Wadenteil 21 eine sich vom Strebenanschluss 46 in Verlängerung der Fersenstrebe 47 erstreckende Wadenstütze 97 aufweist, die einen wadenseitigen Zwischenraum 98 der Wadenstreben 29, 30 im Wesentlichen ausfüllt. Die Wadenstütze 97 ist aufgrund ihrer Formelastizität federnd mit dem Strebenanschluss 46 verbunden, so dass sie bei an einem Unterschenkel angeordneter Stützschalenanordnung 20 mit einer oberen Stützkante 99 federnd an der Wade anliegt. Im Bereich der Stützkante 99 erstreckt sich zwischen der Wadenstütze 97 und den Wadenstreben 29, 30 jeweils ein in seiner Relativanordnung an der Wadenstrebe 29, 30 in unterschiedlichen Befestigungsaufnahmen 100, 101 und 102 positionierbarer Stützenanschlag 103 (**Fig**. **9**). Zur Fixierung in einer Befestigungsaufnahme 100, 101, 102 ist der Stützenanschlag 103 mit einem in die jeweilige Befestigungsaufnahme einführbaren und daraus lösbaren Rastzapfen 104 versehen. An seinem der Wadenstütze 97 zugewandten Ende ist der Stützenanschlag 103 mit einem Eingriffzapfen 105 versehen, der in eine als Langloch 106 ausgebildete Anschlagführung eingreift und somit den Federweg der Wadenstütze 97 begrenzt.

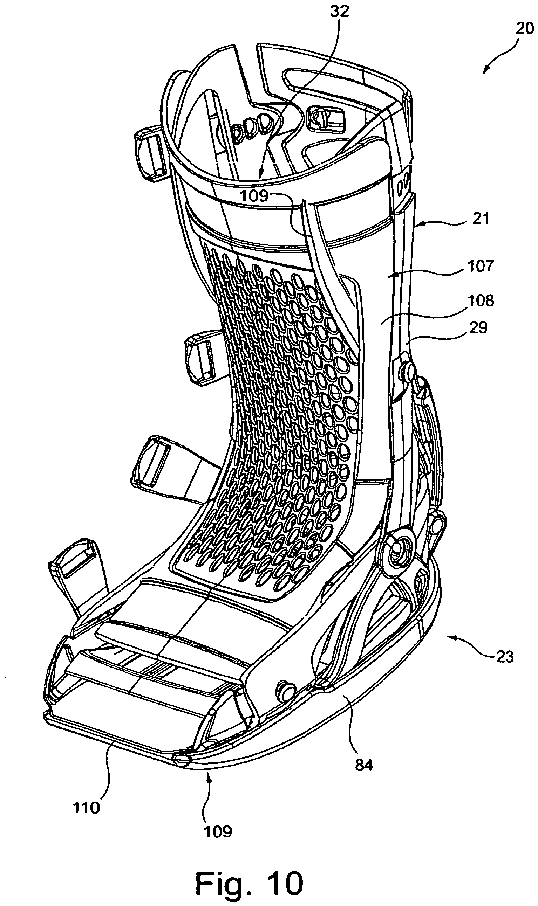

**Fig. 10** zeigt die Stützschalenanordnung 20 mit einer die Einstiegsöffnung 32 schienbeinseitig bis hin zum Mittelfußbereich hin abdeckenden Einstiegsabdeckung 107. Die Einstiegsabdeckung 107 besteht aus einem atmungsaktiven und biegeweichen Material, dem im Gegensatz zum Wadenteil 21 und zum Fußteil 23 der Stützschalenanordnung 20 keine mechanisch stützende Wirkung zukommt. Die Einstiegsabdeckung kann somit hinsichtlich Komfort und hygienischen Ansprüchen optimiert werden.

Wie ferner der Darstellung in **Fig. 10** zu entnehmen ist, können zur Unterstützung der Befestigung der Einstiegsabdeckung 107 an der Stützschalenanordnung 20 Befestigungslaschen 108 um im Vergleich zu den Wadenstreben 29, 30 relativ biegeweich ausgeführte Anschmiegbügel herumgeführt sein, welche formelastisch am Umfang eines im Wadenteil 21 aufgenommenen Unterschenkels anliegen.

Weiterhin ist der **Fig. 10****,** insbesondere im Zusammenhang mit der Darstellung in **Fig. 7****,** zu entnehmen, dass eine Vorderkante 109 der Sohleneinrichtung 84 mit einer stegförmig ausgebildeten Stoßkante 110 belegt sein kann, die im Vergleich zum Material der Sohleneinrichtung 84 eine wesentlich höhere Abrieb- und Schlagfestigkeit aufweisen kann.

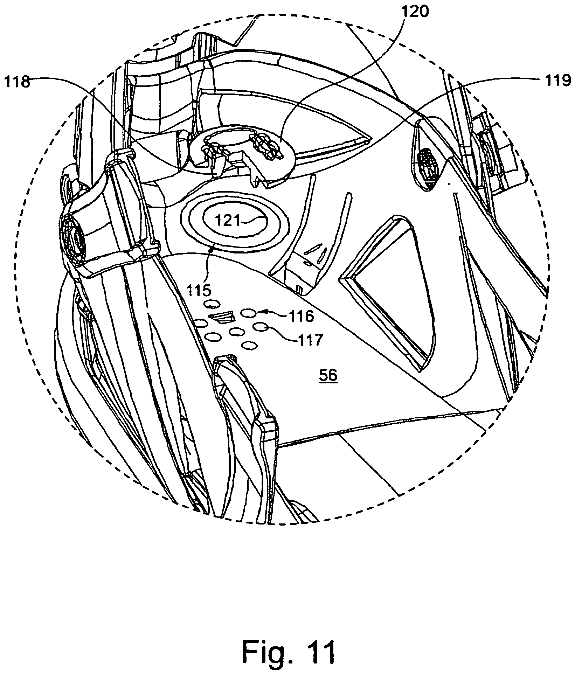

**Fig. 11** zeigt eine weitere Möglichkeit zur Anordnung eines als Federring 115 ausgebildeten Kraftsensors. Hierzu ist im Fersenbereich des Schalenbodens 56 ein Lochmuster 116 mit Löchern 117 zur Aufnahme von Führungszapfen 118, 119 einer mit zumindest zwei Führungszapfen auf ihrer Unterseite versehenen Druckplatte 120 ausgebildet. Die Führungszapfen 118, 119 dienen gleichzeitig zur zentrierenden Positionierung des Federrings 115, der mit seinem Bohrungsrand 121 auf den Führungszapfen 118, 119 radial und axial geführt ist.

Zur Montage des Federrings 115 werden die Führungszapfen 118, 119 der Druckplatte 120 unter zwischenliegender Anordnung des Federrings 115 in das Lochmuster 116 eingesetzt. Bei dem hier abgebildeten Lochmuster 116 ergeben sich im Zusammenwirken mit den insgesamt vier Führungszapfen 118, 119 der Druckplatte 120 insgesamt zwei Montagepositionen, nämlich eine hintere und eine um ein Loch 117 nach vorn versetzte vordere Position, um eine individuelle Anpassung der Montageposition zu ermöglichen.

## Patentansprüche

1. Stützschalenanordnung (20) zur Anordnung an einem Unterschenkel mit einem Fußteil (23) und einem Wadenteil (21), wobei das Wadenteil (21) eine zu einem Fersenteil (48) des Fußteils hin verlaufende Fersenstrebe (47) aufweist, und wobei das Fußteil (23) drehgelenkig an das Wadenteil (21) angeschlossen ist und eine Stützbasis (36) aufweist, die an ihrem fersenseitigen Ende gelenkig mit der Fersenstrebe des Wadenteils verbunden ist,
**dadurch gekennzeichnet,**
**dass** das Wadenteil zwei seitlich einer Einstiegsöffnung (32) des Wadenteils angeordnete, in Längsrichtung des Wadenteils verlaufende Wadenstreben (29, 30) aufweist, und die Fersenstrebe (47) in einem Strebenanschluss (46) mit den Wadenstreben verbunden ist, und dass am Fußteil eine Stützbügelanordnung (24) mit zwei sich U-förmig um einen Fußsohlenbereich herum erstreckenden Bügelstreben (34, 35) angeordnet ist, deren freie Bügelenden (37, 38) paarweise in einem gemeinsamen Drehgelenk (43, 44) auf einer der Fußgelenkachse entsprechenden Gelenkachse (42) drehgelenkig an eine Wadenstrebe angeschlossen sind, und die V-förmig zueinander angestellt über eine gemeinsame Stützbasis (36) miteinander verbunden sind.

2. Stützschalenanordnung nach Anspruch 1,
**dadurch gekennzeichnet,**
**dass** die Stützbasis (36) der Stützbügelanordnung (24) an ihrer Unterseite (82) mit einer Verbindungseinrichtung (113) zur Verbindung mit einer Sohleneinrichtung (84) versehen ist.

3. Stützschalenanordnung nach Anspruch 2,
**dadurch gekennzeichnet,**
**dass** die Stützbasis (36) der Stützbügelanordnung (24) auf ihrer Unterseite (82) mit einer Behelfssohleneinrichtung (87) versehen ist.

4. Stützschalenanordnung nach Anspruch 2 oder 3,
**dadurch gekennzeichnet,**
**dass** die Verbindungseinrichtung als Behelfssohleneinrichtung ausgebildet ist.

5. Stützschalenanordnung nach einem der vorangehenden Ansprüche,
**dadurch gekennzeichnet,**
**dass** die Stützbasis (36) der Stützbügelanordnung (24) auf ihrer Unterseite (82) mit einer Auslöseeinrichtung (88) für einen Kraftsensor (89) versehen ist.

6. Stützschalenanordnung nach einem der vorangehenden Ansprüche,
**dadurch gekennzeichnet,**
**dass** die Stützbügelanordnung (24) als von einer Inlayschale (53) des Fußteils (23) unabhängiges Bauteil ausgebildet ist.

7. Stützschalenanordnung nach Anspruch 6,
**dadurch gekennzeichnet,**
**dass** die Stützbügelanordnung (24) über eine Rastverbindung mit der Inlayschale (53) des Fußteils (23) verbindbar ist.

8. Stützschalenanordnung nach Anspruch 6 oder 7,
**dadurch gekennzeichnet,**
**dass** die Stützbügelanordnung (24) lösbar mit der Inlayschale (53) verbunden ist.

9. Stützschalenanordnung nach einem der vorangehenden Ansprüche 6-8,
**dadurch gekennzeichnet,**
**dass** das Fußteil (23) ein mit der Inlayschale (53) verbindbares Zehenteil (78) aufweist.

10. Stützschalenanordnung nach Anspruch 9,
**dadurch gekennzeichnet,**
**dass** das Zehenteil (78) längsverschiebbar mit der Inlayschale (53) verbunden ist.

11. Stützschalenanordnung nach Anspruch 9 oder 10,
**dadurch gekennzeichnet,**
**dass** das Zehenteil (78) eine gegenüber einem Basisteil (79) zur Verbindung mit der Inlayschale (53) vermittels einer Gelenkeinrichtung (80) verschwenkbare Zehenauflage (81) aufweist.

12. Stützschalenanordnung nach einem der vorangehenden Ansprüche,
**dadurch gekennzeichnet,**
**dass** zur fersenseitigen Verbindung der Stützbügelanordnung (24) des Fußteils (23) mit der Fersenstrebe (47) des Wadenteils (21) eine Beugewinkel-Einstelleinrichtung (49) vorgesehen ist, mit einer an einem Fersenanschluss (48) der Stützbügelanordnung angelenkten Lasche (50), die über einen Führungseingriff an einer an der Fersenstrebe ausgebildeten Führungsschiene (66) in veränderbaren Positionen an der Fersenstrebe fixierbar ist.

13. Stützschalenanordnung nach Anspruch 12,
**dadurch gekennzeichnet,**
**dass** die Lasche (50) einen Laschenanschlag (63) aufweist, der zur beidseitigen Begrenzung eines Verschiebewegs mit zwei in ihrer Relativposition gegenüber der Führungsschiene (66) veränderbar an der Führungsschiene fixierbaren Schienenanschlägen (71, 72) zusammenwirkt.

14. Stützschalenanordnung nach einem der vorangehenden Ansprüche,
**dadurch gekennzeichnet,**
**dass** das Wadenteil (21) eine sich vom Strebenanschluss (46) in einen wadenseitigen Zwischenraum (98) der Wadenstreben (29, 30) erstreckende Wadenstütze (97) aufweist, die sich federnd am Strebenanschluss abstützt.

15. Stützschalenanordnung nach Anspruch 14,
**dadurch gekennzeichnet,**
**dass** die Wadenstütze (97) an ihrem freien Endbereich über längsverschiebbar angeschlossene Anschläge (103) mit den Wadenstreben (29, 30) verbunden ist.

16. Stützschalenanordnung nach Anspruch 15,
**dadurch gekennzeichnet,**
**dass** die Anschläge (103) in unterschiedlichen Positionen an den Wadenstreben (29, 30) fixierbar sind.

## Claims

1. Support shell arrangement (20) for arrangement at a lower leg, having a foot part (23) and a calf part (21), wherein the calf part (21) has a heel strut (47) extending towards a heel part (48) of the foot part, and the foot part (23) is pivotally connected to the calf part (21) and has a support base (36), which at the heel-sided end thereof is articulately connected to the heel strut of the calf part, **characterized in that**
the calf part comprises two calf struts (29, 30) disposed at the side of an entry opening (32) of the calf part and extending into the longitudinal direction of the calf part, and the heel strut (47) is connected to the calf struts in a strut connection (46),
and **in that** a support bow arrangement (24) having two bow struts (34, 35) extending in a U-shape around a sole area is disposed at the foot part, the free strut ends (37, 38) thereof being pivotally connected to a calf strut in pairs in a common pivot joint (43, 44) on a joint axis (42) corresponding to the ankle axis and being connected to one other in a V-shape via a common support base (36).

2. Support shell arrangement according to claim 1,
**characterized in that**
the support base (36) of the support bow arrangement (24), on the lower side (82) thereof, is provided with a connection device (113) for connection with a sole device (84).

3. Support shell arrangement according to claim 2,
**characterized in that**
the support base (36) of the support bow arrangement (24), on the lower side (82) thereof, is provided with an auxiliary sole device (87).

4. Support shell arrangement according to claim 2 or 3,
**characterized in that**
the connection device is designed as an auxiliary sole device.

5. Support shell arrangement according to any of the preceding claims,
**characterized in that**
the support base (36) of the support bow arrangement (24), on the lower side (82) thereof, is provided with an activation device (88) for a force sensor (89).

6. Support shell arrangement according to any of the preceding claims,
**characterized in that**
the support bow arrangement (24) is formed as a component that is independent of an inlay shell (53) of the foot part (23).

7. Support shell arrangement according to claim 6,
**characterized in that**
the support bow arrangement (24) can be connected to the inlay shell (53) of the foot part (23) by means of a snap-fit connection.

8. Support shell arrangement according to claim 6 or 7,
**characterized in that**
the support bow arrangement (24) is detachably connected to the inlay shell (53).

9. Support shell arrangement according to any of the preceding claims 6 to 8,
**characterized in that**
the foot part (23) features a toe part (78) that can be connected to the inlay shell (53).

10. Support shell arrangement according to claim 9,
**characterized in that**
the toe part (78) is connected to the inlay shell (53) so as to be extendable.

11. Support shell arrangement according to claim 9 or 10,
**characterized in that**
the toe part (78) features a toe support (81) being swivelable with respect to a base part (79) by means of an articulated joint (80) for connection with the inlay shell (53).

12. Support shell arrangement according to any of the preceding claims,
**characterized in that**
a bending angle setting device (49) is provided for heel-sided connection of the support bow arrangement (24) of the foot part (23) with the heel strut (47) of the calf part (21) and features a flap (50) articulated to a heel connection (48) of the support bow arrangement and being fixable in variable positions at the heel strut by means of guiding engagement with a guide rail (66) formed at the heel strut.

13. Support shell arrangement according to claim 12,
**characterized in that**
the flap (50) features a flap stop (63) that interacts with two rail stops (71, 72) that can be fixed at the guide rail so as to be variable in their relative positions with respect to the guide rail (66) for limiting the path of displacement on both sides.

14. Support shell arrangement according to any of the preceding claims,
**characterized in that**
the calf part (21) features a calf bracket (97) extending from the strut connection (46) into a calf-sided gap (98) of the calf struts (29, 30) and being resiliently supported against the strut connection.

15. Support shell arrangement according to claim 14,
**characterized in that**
the calf bracket (97), at the free terminal region thereof, is connected to the calf struts (29, 30) via stops (103) that are connected so as to be longitudinally displaceable.

16. Support shell arrangement according to claim 15,
**characterized in that**
the stops (103) can be fixed at the calf struts (29, 30) in various positions.

## Revendications

1. Dispositif de coque de soutien (20) pour mise en place contre une jambe, comportant une partie pied (23) et une partie mollet (21), la partie mollet (21) comportant un étai de talon (47) s'étendant vers une partie talon (48) de la partie pied et la partie pied (23) étant reliée d'une manière pivotante avec la partie mollet (21) et présentant une base de soutien (36) qui, à son extrémité du côté talon, est reliée d'une manière articulée avec l'étai de talon de la partie mollet,
**caractérisé en ce que**
la partie mollet comporte deux étais de mollet (29, 30) disposés latéralement par rapport à une ouverture d'entrée (32) de la partie mollet et s'étendant dans la direction longitudinale de la partie mollet, et l'étai de talon (47) est relié aux étais de mollet à l'aide d'un raccordement d'étais (46), et un système d'étrier de soutien (24) comportant deux étais d'étrier (34, 35) s'étendant en forme de U autour d'une zone de talon est disposé à la partie pied, les extrémités libres (37, 38) des étais d'étrier étant reliées d'une manière pivotante, par paires, avec un étai de mollet dans une articulation à charnière (43, 44) commune sur un axe d'articulation (42) correspondant à l'axe d'articulation du pied, et étant raccordées l'une à l'autre en forme de V par l'intermédiaire d'une base de soutien (36) commune.

2. Dispositif de coque de soutien selon la revendication 1,
**caractérisé en ce que**
la base de soutien (36) du système d'étrier de soutien (24) sur son côté inférieur (82) est munie d'un dispositif de raccordement (113) afin d'être raccordée à un dispositif de semelle (84).

3. Dispositif de coque de soutien selon la revendication 2,
**caractérisé en ce que**
la base de soutien (36) du système d'étrier de soutien (24) sur son côté inférieur (82) est munie d'un dispositif de semelle auxiliaire (87).

4. Dispositif de coque de soutien selon la revendication 2 ou 3,
**caractérisé en ce que**
le dispositif de raccordement est conçu en tant que dispositif de semelle auxiliaire.

5. Dispositif de coque de soutien selon l'une quelconque des revendications précédentes,
**caractérisé en ce que**
la base de soutien (36) du système d'étrier de soutien (24) sur son côté inférieur (82) est munie d'un mécanisme d'actionnement (88) pour un capteur de force (89).

6. Dispositif de coque de soutien selon l'une quelconque des revendications précédentes,
**caractérisé en ce que**
le système d'étrier de soutien (24) est conçu en tant qu'un composant qui est indépendant d'une coque d'insert (53) de la partie pied (23).

7. Dispositif de coque de soutien selon la revendication 6,
**caractérisé en ce que**
le système d'étrier de soutien (24) peut être relié avec la coque d'insert (53) de la partie pied (23) à l'aide d'une connexion à encliquetage.

8. Dispositif de coque de soutien selon la revendication 6 ou 7,
**caractérisé en ce que**
le système d'étrier de soutien (24) est relié de façon amovible avec la coque d'insert (53).

9. Dispositif de coque de soutien selon l'une quelconque des revendications précédentes 6 à 8,
**caractérisé en ce que**
la partie pied (23) présente une partie orteil (78) qui peut être reliée avec la coque d'insert (53).

10. Dispositif de coque de soutien selon la revendication 9,
**caractérisé en ce que**
la partie orteil (78) est reliée avec la coque d'insert (53) de façon à être déplaçable en direction longitudinale.

11. Dispositif de coque de soutien selon la revendication 9 ou 10,
**caractérisé en ce que**
la partie orteil (78) présente un support d'orteil (81) qui est pivotable par rapport à une partie de base (79) par l'intermédiaire d'un mécanisme d'articulation (80) afin d'être raccordé à la coque d'insert (53).

12. Dispositif de coque de soutien selon l'une quelconque des revendications précédentes,
**caractérisé en ce qu'**
un dispositif de réglage d'angle de flexion (49) est prévu pour relier, du côté du talon, le système d'étrier de soutien (24) de la partie pied (23) avec l'étai de talon (47) de la partie mollet (21) et présente une patte (50) qui est articulée à un raccordement de talon (48) du système d'étrier de soutien et qui peut être fixée à l'étai de talon dans différentes positions en venant en engrenage de guidage avec un rail de guidage (66) formé à l'étai de talon.

13. Dispositif de coque de soutien selon la revendication 12,
**caractérisé en ce que**
la patte (50) présente une butée de patte (63) interagissant avec deux butées de rail (71, 72) pouvant être fixées au rail de guidage (66) de façon variable dans leurs positions relatives par rapport au rail de guidage (66) pour délimiter le chemin de déplacement des deux côtés.

14. Dispositif de coque de soutien selon l'une quelconque des revendications précédentes,
**caractérisé en ce que**
la partie mollet (21) présente un soutien de mollet (97) s'étendant à partir du raccordement d'étais (46) dans un écartement (98) des étais de mollet (29, 30) situé du côté mollet et s'appuyant élastiquement contre le raccordement d'étais.

15. Dispositif de coque de soutien selon la revendication 14,
**caractérisé en ce que**
le soutien de mollet (97) dans sa zone terminale libre est relié aux étais de mollet (29, 30) via des butées (103) qui sont raccordées de manière à être déplaçable en direction longitudinale.

16. Dispositif de coque de soutien selon la revendication 15,
**caractérisé en ce que**
les butées (103) peuvent être fixées aux étais de mollet (29, 30) dans différentes positions.
